(19) Europäisches Patentamt
European Patent Office
Office européen des brevets

(11) Veröffentlichungsnummer : **0 305 329 B1**

(12) # EUROPÄISCHE PATENTSCHRIFT

(45) Veröffentlichungstag der Patentschrift : **02.10.91 Patentblatt 91/40**

(51) Int. Cl.$^5$ : **C09B 5/08**

(21) Anmeldenummer : **88810558.2**

(22) Anmeldetag : **16.08.88**

(54) **Verfahren zur Herstellung einer N-alkylierten Bipyrazolanthronverbindung.**

(30) Priorität : **24.08.87 CH 3230/87**

(43) Veröffentlichungstag der Anmeldung : **01.03.89 Patentblatt 89/09**

(45) Bekanntmachung des Hinweises auf die Patenterteilung : **02.10.91 Patentblatt 91/40**

(84) Benannte Vertragsstaaten : **CH DE FR GB IT LI**

(56) Entgegenhaltungen : **CHEMICAL ABSTRACTS, Band 79, Nr. 16, 22. October 1973, Seite 66, Ref.Nr. 93443r, Columbus, Ohio, US; & CS-A-14 77 80 (J. ARIENT) 15-03-1973**

(73) Patentinhaber : **CIBA-GEIGY AG
Klybeckstrasse 141
CH-4002 Basel (CH)**

(72) Erfinder : **Hildebrand, Rainer, Dr.
Haltinger Pfad 5
W-7850 Lörrach (DE)**

EP 0 305 329 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung einer N-alkylierten 2,2'-Bipyrazolanthronverbindung der Formel

(1) 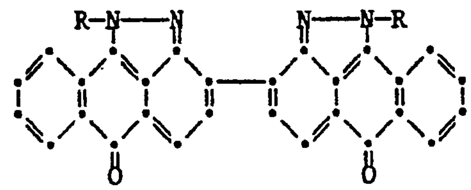

worin R $C_1$-$C_8$-Alkyl bedeutet, das dadurch gekennzeichnet ist, dass man 1,9-Pyrazolanthron der Formel

(2) 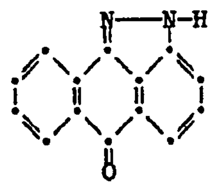

in Gegenwart eines Alkalimetallhydroxides und eines Alkanols mit 1 bis 5 Kohlenstoffatomen und bei erhöhter Temperatur dimerisiert und das erhaltene Zwischenprodukt der Formel

(3) 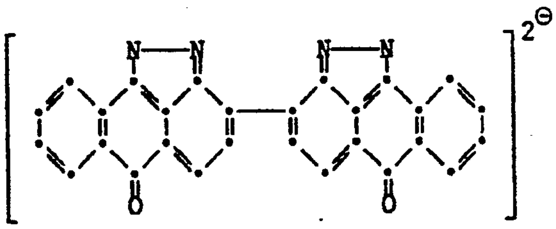 2 $Me^{\oplus}$

worin Me ein Alkalimetall ist, mit einem Alkylhalogenid, welches den Alkylrest R im molaren Verhältnis 1 : 2 einführt und in Gegenwart eines Alkylenglykols oder eines $C_1$-$C_4$-Alkylethers davon als Katalysator umsetzt.

R ist vorzugsweise $C_1$-$C_5$-Alkyl, insbesondere Methyl oder vor allem Ethyl.

Bei dem Alkalimetallhydroxid handelt es sich z.B. um Kaliumhydroxid, Natriumhydroxid oder auch um ein Gemisch aus Kaliumhydroxid und Natriumhydroxid.

Das Gewichtsverhältnis von Alkalimetallhydroxid zur als Ausgangsstoff verwendeten 1,9-Pyrazolanthronverbindung beträgt vorzugsweise zwischen 5 : 1 und 0,9 : 1, vorzugsweise von 3,5 : 1 bis 2 : 1.

Das benutzte Alkanol ist bevorzugt ein Alkohol mit einem Siedepunkt von < 120°C. In Frage kommen insbesondere Methanol, Ethanol, n-Propanol, Isopropanol, n-Butanol, 2-Butanol, Isobutanol, tert.Butanol oder Isopentanol. In manchen Fällen kann auch n-Pentanol verwendet werden.

Besonders bevorzugt verwendet man Methanol, Ethanol oder Isopropanol. Es können auch Mischungen der Alkanole verwendet werden.

Das Verfahren wird z.B. mit der 1- bis 4-fachen Gewichtsmenge Alkalimetallhydroxid, bezogen auf das Alkanol, durchgeführt.

Besonders vorteilhaft ist es, wenn man eine Mischung aus Kaliumhydroxid und Ethanol verwendet. Dabei entsteht eine Kaliumhydroxid-Ethanolschmelze.

Die Reaktionstemperatur zur Dimerisierung der Pyrazolanthronverbindung wird von der gebildeten Alkalischmelze beeinflusst. Man arbeitet vorzugsweise bei einer Temperatur von 110° bis 180°C, vorzugsweise 130 bis 150°C. Nach der Alkalischmelze wird das Reaktionsprodukt einer Luftoxidation, die in der Regel bei 60° bis 95° erfolgt, unterworfen, worauf das 2,2'-Bipyrazolanthron in Form eines Alkalimetallsalzes erhalten wird.

Die nachträgliche Alkylierung erfolgt vorzugsweise ohne Isolierung des Zwischenproduktes. Sie wird mit einem Alkylhalogenid, wie z.B. Iodid, Bromid oder Chlorid durchgeführt.

Das Alkylhalogenid wird in äquivalenten Mengen zur Einführung der zwei Alkylreste R benötigt. Ein geringer Ueberschuss des Alkylhalogenides über die Molmenge der Bipyrazolanthronverbindung wird gewöhnlich

angewendet. Nicht umgesetztes Alkylhalogenid kann anschliessend durch Abdestillieren entfernt oder auch zu Alkanol hydrolisiert werden.

Die Alkylierung kann bei 10 bis 100°C, vorzugsweise 10 bis 80°C oder unter der Siedetemperatur des Alkylhalogenides durchgeführt werden. Man kann auch unter Druck bei erhöhter Temperatur im Autoklaven z.B. bis zu 200°C arbeiten. Die Reaktion ist häufig exotherm und kann gegebenenfalls durch Kühlen gesteuert werden.

Als Alkylhalogenide eignen sich beispielsweise Methylchlorid, Methylbromid, Ethylchlorid, Ethylbromid, Propylchlorid, Propylbromid, 3-Methylbutylbromid oder Butylbromid. Bevorzugt ist Ethylbromid.

Die Alkylierung wird in Gegenwart eines Katalysators, der ein Alkylenglykol oder ein Mono-$C_1$-$C_4$-Alkylether des Alkylenglykols ist, durchgeführt, wobei in der Regel 0,5 bis 10 Gewichtsprozent, vorzugsweise 1 bis 5 Gewichtsprozent, bezogen auf Bipyrazolanthron, eingesetzt werden.

Beispiele von Katalysatoren sind Ethylenglykol, Propylenglykol, Diethylenglykol, Dipropylenglykol, Polyethylenglykole, Polypropylenglykole, Ethylenglykolmonomethylether, Ethylenglykolmonoethylether, Ethylenglykolmonobutylether, Diethylenglykolmonomethylether oder -monoethylether, Triethylenglykolmonomethylether. Auch Mischungen dieser Alkylenglykole können verwendet werden. Bevorzugter Katalysator ist Polyethylenglykol mit einem durchschnittlichen Molekulargewicht von 300 bis 1000 oder 300 bis 650, besonders 400-450.

Nach Beendigung der Alkylierung wird die N-alkylierte Bipyrazolanthronverbindung auf übliche Weise isoliert z.B. durch Filtrieren oder Dekantieren der erhaltenen Suspension, Waschen des Produktes und Trocknen.

Gewünschtenfalls kann die erhaltene N-alkylierte Bipyrazolanthronverbindung einem Reinigungsprozess zur Entfernung nicht erwünschter Isomerenverbindungen durch Lösungsmittelextraktion unterworfen werden. Hierfür eignen sich als Lösungsmittel Ethylacetat, Aceton, Methylenchlorid, Dimethylacetamid, Ameisensäure, Amylalkohol, vorteilhafterweise Toluol, Xylol, Chlorbenzole, wie Dichlorbenzol oder Trichlorbenzol oder besonders Nitrobenzol.

Die erhaltene N-alkylierte Bipyrazolanthronverbindung ist ein Küpenfarbstoff zum Färben oder Bedrucken von Fasern aus natürlicher oder regenerierter Cellulose in Gegenwart von Reduktionsmitteln, wie z.B. Dithionit.

Aus Chemical Abstracts, Band 79, Nr. 16, Seite 66, Nr. 93443r sind Tetramethyl-bis-pyrazolanthronverbindungen bekannt, die durch Dimerisierung von 5,8-Dimethylpyrazolanthron mit anschliessender Alkylierung hergestellt werden. Die Alkylierung erfolgt bei 140-145°C in Dichlorbenzol mit p-Toluolsulfonsäuremethylester oder -isopropylester und in Gegenwart von Kaliumcarbonat.

Durch das erfindungsgemässe Verfahren wird es möglich die N-alkylierte Bipyrazolanthronverbindungen der Formel (1) besonders N-ethyliertes 2,2'-Bipyrazolanthron in besonders einfacher Weise in sehr guten Ausbeuten herzustellen. Man kann die Bipyrazolanthron-Metallsalzherstellung und anschliessende Umsetzung mit einem Alkylhalogenid zu einem Arbeitsgang zusammenziehen. Man kann somit die erfindungsgemässe Alkylierung wesentlich rascher und ökonomischer als z.B. mit einem Dialkylsulfat durchführen. Das neue Verfahren ermöglicht also eine sehr wesentliche Arbeits-und Zeitersparnis, fordert weniger Energie und liefert zudem einen Küpenfarbstoff in sehr guter Ausbeute und hoher Reinheit. Im folgenden Beispiel beziehen sich Prozentsätze auf das Gewicht.

Beispiel : 275,5 g Kaliumhydroxid und 76,6 g Ethanol werden auf 130°C aufgeheizt. Hierauf werden 74,9 g 1,9-Pyrazolanthron portionenweise im Verlaufe von 30 Minuten zugegeben. Das Gemisch wird bei 140-145°C 2 1/2 Stunden nachgerührt. Alsdann wird das Reaktionsprodukt auf 125°C abgekühlt und man lässt 200 ml Wasser zutropfen. Dabei destilliert man Ethanol/Wasser ab. Hiernach wird die wässerige Suspension durch Einblasen von Luft 3 Stunden bei 80°C oxidiert und schliesslich auf Raumtemperatur abgekühlt. Man erhält das 2,2'-Bipyrazolanthron-Kaliumsalz in Suspension.

Ohne Isolierung wird eine Mischung von 5 g Polyethylenglykol (MG 400) und 87,6 g Ethylbromid zugegeben. Nach 1 Stunde bei 25°C erwärmt man auf 33°C und rührt 15 Stunden, bis die Reaktion vollständig ist. Die Suspension wird filtriert, worauf der Rückstand mit 2 Liter Wasser gewaschen und im Vakuum bei 80°C getrocknet wird.

Das Rohprodukt wird einer Lösungsmittelextraktion unterzogen, wobei 62,7 g eines isomerenreinen Farbstoffes der Formel

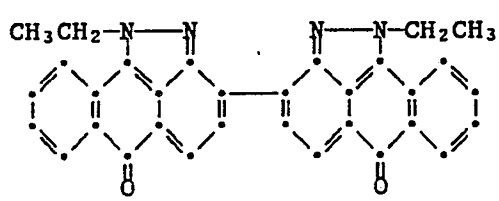

mit einer Ausbeute von 80% der Theorie anfällt.

Verfährt man wie im Beispiel beschrieben, so wird unter Verwendung der in der Tabelle aufgeführten Alkylierungsmittel, Katalysatoren und Temperaturen das entsprechende alkylierte Bipyrazolanthron mit der in der letzten Spalte der Tabelle angegebenen Ausbeute erhalten.

Tabelle

| Bsp. | Alkylierungsmittel | Katalysator | Temp./°C | Ausbeute |
|---|---|---|---|---|
| A | 60 ml Ethylbromid | Polyethylen-glykol-300 | 33 | 66,5 g; 79 % |
| B | 60 ml Ethylbromid | Ethylenglykol-monoethylether | 33 | 65,2 g; 77 % |
| C | 60 ml Ethylbromid | Polyethylenglykol-methylether 750 | 33 | 64,1 g; 75 % |
| D | 50 ml Methyljodid | Polyethylen-glykol-400 | 34 | 52,4 g; 66 % |
| E | 73 ml n-Propylbromid | Polyethylen-glykol-400 | 65 | 68,0 g; 76 % |
| F | 100 ml 3-Methylbutyl-bromid | Polyethylen-glykol-400 | 80 | 68,9 g; 70 % |
| G | 60 ml Ethylbromid | Polyethylen-glykol 1000 | 33 | 59,7 g; 71 % |
| H | 140 ml Bromoctan | Polyethylen-glykol-400 | 100 | 65 g; 71 % |

## Patentansprüche

1. Verfahren zur Herstellung eine N-alkylierten Bipyrazolanthronverbindung der Formel

(1)

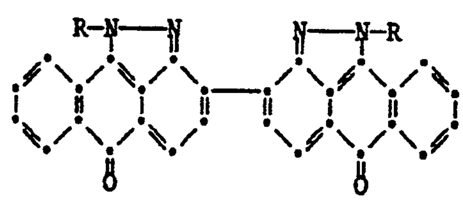

worin R $C_1$-$C_8$-Alkyl bedeutet, dadurch gekennzeichnet, dass man 1,9-Pyrazolanthron der Formel

(2) 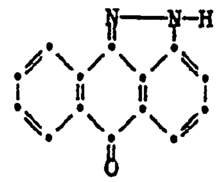

in Gegenwart eines Alkalimetallhydroxides und eines Alkanols mit 1 bis 5 Kohlenstoffatomen und bei erhöhter Temperatur dimerisiert und das erhaltene Zwischenprodukt der Formel

(3) 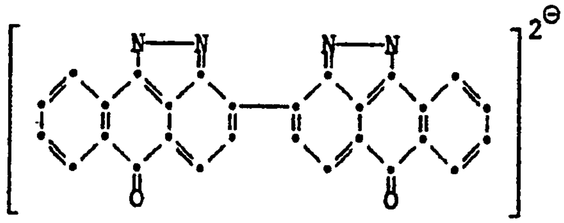 2 $Me^{\oplus}$

worin Me ein Alkalimetall ist, mit einem Alkylhalogenid, welches den Alkylrest R im molaren Verhältnis 1 : 2 einführt und in Gegenwart eines Alkylenglykols oder eines $C_1$-$C_4$-Alkylethers davon als Katalysator umsetzt.

2. Verfahren gemäss Anspruch 1, dadurch gekennzeichnet, dass in Formel (1) R Methyl oder besonders Ethyl bedeutet.

3. Verfahren gemäss einem der Ansprüche 1 und 2, dadurch gekennzeichnet, dass man eine Mischung aus Kaliumhydroxid und Ethanol verwendet.

4. Verfahren gemäss einem der Ansprüche 1 bis 3, dadurch gekennzeichnet, dass die Dimerisierung bei einer Temperatur von 110° bis 180°C durchgeführt wird.

5. Verfahren gemäss einem der Ansprüche 1 bis 4, dadurch gekennzeichnet, dass die Alkylierung ohne Isolierung des Zwischenproduktes erfolgt.

6. Verfahren gemäss einem der Ansprüche 1 bis 5, dadurch gekennzeichnet, dass man die Alkylierung mit einem Alkylbromid, besonders Ethylbromid durchführt.

7. Verfahren gemäss einem der Ansprüche 1 bis 6, dadurch gekennzeichnet, dass man die Umsetzung mit dem Alkylhalogenid in Gegenwart von Polyethylenglykol durchführt.

8. Verfahren gemäss Anspruch 7, dadurch gekennzeichnet, dass das Polyethylenglykol ein durchschnittliches Molekulargewicht von 300 bis 1000 hat.

**Claims**

1. A process for the preparation of an N-alkylated bipyrazoleanthrone compound of formula

(1) 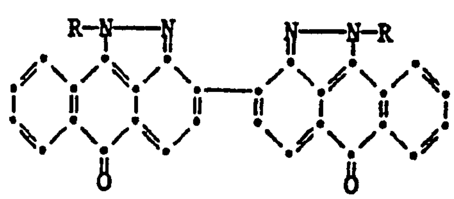

in which R is $C_1$-$C_8$alkyl, which comprises dimerising 1,9-pyrazoleanthrone of formula

(2)

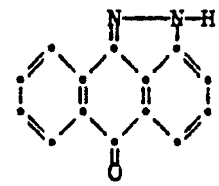

in the presence of an alkali metal hydroxide and an alkanol of 1 to 5 carbon atoms and at elevated temperature, and reacting the resultant intermediate of formula

(3)

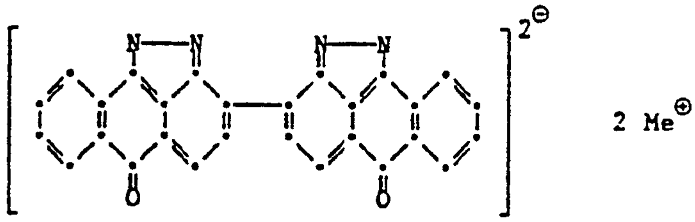

in which Me is an alkali metal, with an alkyl halide which introduces the alkyl radical R, in the molar ratio 1 : 2, and in the presence of an alkylene glycol or a $C_1$-$C_4$ alkyl ether thereof as catalyst.

2. A process according to claim 1, wherein R in formula (1) is methyl or, in particular, ethyl.

3. A process according to either of claims 1 and 2, wherein a mixture of potassium hydroxide and ethanol is used.

4. A process according to any one of claims 1 to 3, wherein the dimerisation is carried out at a temperature from 110° to 180°C.

5. A process according to any one of claims 1 to 4, wherein the alkylation is carried out without isolation of the intermediate.

6. A process according to any one of claims 1 to 5, wherein the alkylation is carried out with an alkyl bromide, in particular ethyl bromide.

7. A process according to any one of claims 1 to 6, wherein the reaction with the alkyl halide is carried out in the presence of polyethylene glycol.

8. A process according to claim 7, wherein the polyethylene glycol has an average molecular weight of 300 to 1000.

**Revendications**

1. Procédé de préparation d'un composé bis-pyrazolanthrone N-alkylé, de formule

(1)

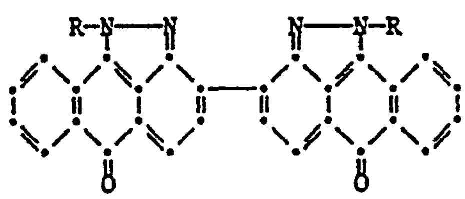

dans laquelle R représente un groupe alkyle en $C_{1-8}$, caractérisé en ce que l'on dimérise, à température élevée, une 1,9-pyrazolanthrone de formule

(2) 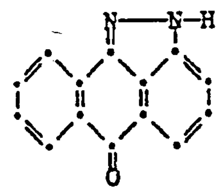

en présence d'un hydroxyde de métal alcalin et d'un alcanol comportant de 1 à 5 atomes de carbone, et que l'on fait réagir le produit intermédiaire obtenu, de formule

(3) 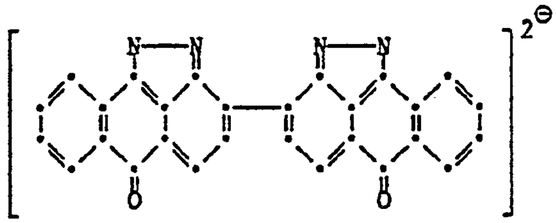 $2\ Me^{\oplus}$

dans laquelle Me est un métal alcalin, avec un halogénure d'alkyle, qui introduit le reste alkyle R selon un rapport molaire de 1 : 2, en présence, comme catalyseur, d'un alkylèneglycol ou d'un éther d'alkyle en $C_{1-4}$ de ce dernier.

2. Procédé selon la revendication 1, caractérisé en ce que, dans la formule (1), R représente un groupe méthyle ou, surtout, éthyle.

3. Procédé selon une des revendications 1 et 2, caractérisé en ce que l'on utilise un mélange d'hydroxyde de potassium et d'éthanol.

4. Procédé selon une des revendications 1 à 3, caractérisé en ce que la dimérisation est réalisée à une température de 110° à 180°C.

5. Procédé selon une des revendications 1 à 4, caractérisé en ce que l'alkylation se déroule sans isolement du produit intermédiaire.

6. Procédé selon une des revendications 1 à 5, caractérisé en ce qu'on réalise l'alkylation avec un bromure d'alkyle, en particulier le bromure d'éthyle.

7. Procédé selon une des revendications 1 à 6, caractérisé en ce qu'on réalise la réaction avec l'halogénure d'alkyle en présence de polyéthylèneglycol.

8. Procédé selon la revendication 7, caractérisé en ce que le polyéthylèneglycol a une masse moléculaire moyenne de 300 à 1000.